# EUROPEAN PATENT SPECIFICATION

(11) **EP 1 602 372 B1**
(45) Date of publication and mention of the grant of the patent: **31.08.2016**
(21) Application number: 04707294.7
(22) Date of filing: 02.02.2004
(51) Int. Cl.: A61K 31/202, A61K 31/232, A61P 7/04, A61P 9/00, A61P 9/10, A61P 43/00

(54) **DRUG FOR IMPROVING PROGNOSIS FOR SUBARACHNOID HEMORRHAGE**
ARZNEIMITTEL ZUR BESSEREN PROGNOSE FÜR SUBARACHNOIDE BLUTUNG
MEDICAMENT AMELIORANT LE PRONOSTIC DE L'HEMORRAGIE SOUS ARACHNOIDIENNE

(30) Priority: 07.02.2003 JP 2003031144
(43) Date of publication of application: 07.12.2005
(73) Proprietor: MOCHIDA PHARMACEUTICAL CO., LTD., Shinjuku-ku Tokyo 160-8515 (JP)
(72) Inventor: KOBAYASHI, Sei, Yamaguchi 7550036 (JP); SUZUKI, Michiyasu, Yamaguchi 7550096 (JP); IKEDA, Norio, Yamaguchi 7550151 (JP)
(74) Representative: Grünecker Patent- und Rechtsanwälte PartG mbB
(86) International application number: PCT/JP2004/000989
(87) International publication number: WO 2004/069238

(56) References cited:
- JP-A- 2002 363 095
- DATABASE BIOSIS [Online] BIOSCIENCES INFORMATION SERVICE, PHILADELPHIA, PA, US; 1985, SHIBUYA T ET AL: "INFLUENCE ON THE PREVENTIVE EFFECT OF THE EICOSAPENTAENOIC-ACID ON CEREBROVASCULAR DISORDERS" XP09137568 Database accession no. PREV198529047932 & JOURNAL OF NIPPON MEDICAL SCHOOL, vol. 52, no. 2, 1985, pages 222-224, ISSN: 0048-0444
- KATAYAMA YASUO ET AL: "Effect of long-term administration of ethyl eicosapentate (EPA-E) on local cerebral blood flow and glucose utilization in stroke-prone spontaneously hypertensive rat (SHRSP)" BRAIN RESEARCH, vol. 761, no. 2, 1997, pages 300-305, XP002596719 ISSN: 0006-8993
- KATAYAMA Y ET AL: "Effect of ethyl icosapentate (EPA) on cerebral vascular disease" MYAKKANGAKU - JOURNAL OF JAPANESE COLLEGE OF ANGIOLOGY, NIHON MYAKKAN GAKKAI, TOKYO, JP, vol. 34, no. 8, 1 January 1994 (1994-01-01), pages 477-487, XP003024416 ISSN: 0387-1126
- CHAN R C ET AL: "The role of the prostacyclin-thromboxane system in cerebral vasospasm following induced subarachnoid hemorrhage in the rabbit" MEDLINE, 1 December 1984 (1984-12-01), XP009137639
- KOBAYASHI TETSUYUKI ET AL: "Assessment of the possible adverse effects of oils enriched with n-3 fatty acids in rats; peroxisomal proliferation, mitochondrial dysfunctions and apoplexy" JOURNAL OF NUTRITIONAL BIOCHEMISTRY, vol. 7, no. 10, 1996, pages 542-548, XP002596720 ISSN: 0955-2863
- YASUGI T., ET AL: 'Eicosapentaenoic acid, docosahexaenoix acid and cerebral hemorrhage: EPA and DHA loading study in spontaneously hypertensive rats' ESSENT FATTY ACIDS EICOSANOIDS, INVITED PAP. INT. CONGR. no. 3RD, 1992, pages 339 - 344, XP002976482
- KROMANN N ET AL: "EPIDEMIOLOGICAL STUDIES IN THE UPERNAVIK DISTRICT, GREENLAND", ACTA MEDICA SCANDINAVICA, ALMQVIST & WIKSELL PERIODICAL CO., STOCKHOLM, SE, vol. 208, no. 5, 1 January 1980 (1980-01-01), pages 401-406, XP000901977, ISSN: 0001-6101
- Dr G C Viberti ET AL: "H MOSTATIC FUNCTION AND PLATELET POLYUNSATURATED FATTY ACIDS IN ESKIMOS", The Lancet, vol. 314, no. 8140, 1 September 1979 (1979-09-01), pages 433-435, XP055171248, ISSN: 0023-7507
- DYERBERG J ET AL: "EICOSAPENTAENOIC ACID AND PREVENTION OF THROMBOSIS AND ATHEROSCLEROSIS?", THE LANCET, THE LANCET PUBLISHING GROUP, GB, vol. 2, no. 8081, 15 July 1978 (1978-07-15), pages 117-119, XP009031790, ISSN: 0140-6736, DOI: 10.1016/S0140-6736(78)91505-2

## Description

The present invention relates to a drug for use in improving the prognosis of a patient with subarachnoid hemorrhage which contains as an active ingredient at least one member selected from the group consisting of an eicosapentaenoic acid (hereinafter, abbreviated as "EPA") and pharmaceutically acceptable salts and esters thereof.

Subarachnoid hemorrhage is due to the rupture of cerebral aneurysm and refers to the state that hemorrhage has occurred in the subarachnoid cavity. In this country, 12 out of 100,000 people a year have suffered subarachnoid hemorrhage. It is an extremely serious disorder, as has been reported that about 20 to 40% of them would die out.

In addition, as complications of subarachnoid hemorrhage, there are known rebleeding, cerebral vasospasm, hydrocephalus, and the like. As a clinical treatment for those complications, a surgical operation such as clipping or coiling is conducted for preventing rebleeding. In addition, a surgical operation such as ventricular drainage or VP shunt has been performed for the appearance of hydrocephalus.

The detailed occurrence mechanism of cerebral vasospasm has been less well understood and in the present circumstances there is no definitive therapeutic method. However, for preventing a spasm, an intraspinal injection of nicardipine hydrochloride (indwelling drainage in the cisterna) or the like is performed. Besides, when the spasm has occurred, a superselective intraarterial injection treatment with papaverine hydrochloride or the like is performed. As a drug for improving subarachnoid hemorrhage-postoperative cerebral vasospasm and corresponding cerebral ischemia symptom, an intravenous injection of Eril injection (generic name: fasudil hydrochloride) is used clinically.

On the other hand, in conjunction with cerebral vasospasm, EPA has been disclosed in publications. For example, in the literatures cited in JP-A 2001-261556, in which an inhibitor for an abnormal contraction of a smooth muscle, which contains EPA as an active ingredient, is disclosed, the above cerebral vasospasm is exemplified as one of the disorders to be caused by the abnormal contraction of the smooth muscle.

However, as far as the inventors of the present invention know, there is no report describing that EPA may enhance the action of improving the prognosis of subarachnoid hemorrhage, particularly its use in clinical application, and increasing a recovery rate as well as a survival rate in clinical application.

Subarachnoid hemorrhage is an extensively serious disorder of high lethality, so that such a disease may turn one into a vegetable state or often leave aftereffects in many cases even though one fortunately was not led to death. When subarachnoid hemorrhage was developed, as described above, a therapeutic agent for preventing or inhibiting cerebral vasospasm is mainly used as a treatment for the complication in addition to surgical operations for rebleeding and hydrocephalus. However, results of the therapeutic agents and therapeutic methods which are currently used in clinical application, particularly prognostic improvement effects, are insufficient. Therefore, the development of a drug having an effect of improving prognosis has been expected.

Accordingly, an object of the present invention is to provide a drug that improves the prognosis of a patient with subarachnoid hemorrhage, which has been under the conditions described above, and enhances a recovery rate or a survival rate with respect to such a disease.

The inventors of the present invention have intensely studied a drug for improving the prognosis of a patient with subarachnoid hemorrhage and enhancing a recovery rate and a survival rate thereof. Finally, the inventors of the present invention have completed the present invention by finding out that the drug of the present invention containing EPA as an active ingredient has the above actions, in particular, an extremely higher lifesaving effect.

As described above, among the complications of subarachnoid hemorrhage, it is known that EPA has an inhibitory effect on cerebral vasospasm (on abnormal contraction of the smooth muscle). However, a cause to lead to death or critical state after subarachnoid hemorrhage is not always due to the cerebral vasospasm which is one of the complications thereof.

The present invention provides a drug for use in improving the prognosis of a patient with subarachnoid hemorrhage, containing as an active ingredient at least one member selected from the group consisting of eicosapentaenoic acid, pharmaceutically acceptable salts and esters thereof, wherein the drug is administered orally or through a stomach tube, and wherein the term "improving prognosis" designates an improvement in the disease state or lethality of subarachnoid hemorrhage.

In another embodiment, the present invention provides the use of a drug for the preparation of a medicament for improving the prognosis of a patient with subarachnoid hemorrhage, containing as an active ingredient at least one member selected from the group consisting of eicosapentaenoic acid, pharmaceutically acceptable salts and esters thereof, wherein the drug is administered orally or through a stomach tube, and wherein the term "improving prognosis" designates an improvement in the disease state or lethality of subarachnoid hemorrhage.

In another embodiment, the present invention provides a drug for use in improving the prognosis of a patient with subarachnoid hemorrhage, containing as an active ingredient at least one member selected from the group consisting of eicosapentaenoic acid, pharmaceutically acceptable salts and esters thereof, wherein the drug is administered orally or through a stomach tube after an operation involved in subarachnoid hemorrhage, and wherein the term "improving prognosis" designates an improvement in the disease state or lethality of subarachnoid hemorrhage.

In another embodiment, the present invention provides the use of a drug for the preparation of a medicament for improving the prognosis of a patient with subarachnoid hemorrhage, containing as an active ingredient at least one member selected from the group consisting of eicosapentaenoic acid, pharmaceutically acceptable salts and esters thereof, wherein the drug is administered orally or through a stomach tube after an operation involved in subarachnoid hemorrhage, and wherein the term "improving prognosis" designates an improvement in the disease state or lethality of subarachnoid hemorrhage.

In a preferred embodiment, the improvement in the disease state is an improvement of recovery rate according to the Glasgow Outcome Scale.

In another preferred embodiment, the drug is administered in combination with a cholagogue drug through a stomach tube.

Hereinafter, the present invention will be described in detail. The term "subarachnoid hemorrhage" as used herein means a hemorrhage state in a subarachnoid cavity. The rupture of cerebral aneurysm occupies about 60-80% of the causes. In addition other causes include head injury, rupture of cerebral arteriovenous malformation, and moyamoya disease of children.

The term "prognosis improvement" as used herein means an improvement in disease state or lethality in comparison with the conventional therapy. For instance, as a specific example, such an improvement can be found in a clinical parameter such as the Glasgow Outcome Scale (GOS) in comparison with the conventional therapy.

EPA to be used in the present invention may be one commercially available or any of those obtained by purifying fish oils, EPA-producing bacterial cells and the culture medium thereof by any of known methods such as a continuous distillation method, a urea adduct method, a liquid chromatography method, a supercritical fluid chromatography method, and a combination thereof. Besides, if necessary, the resulting EPA may be subjected to an esterification treatment to make it into an ester such as alkyl ester, including ethyl ester, or glyceride. In addition, it may be prepared in a salt form with an inorganic base such as a sodium salt or a potassium salt; an organic base such as a benzylamine salt or a diethylamine salt; or a basic amino acid such as an arginine salt or a lysine salt.

In the present invention, EPA includes the above salts and esters in addition to a free fatty acid unless otherwise specified. where EPA is administered to a human or an animal, preferable is of pharmaceutically acceptable. Of those, EPA is preferably used in an ester form of ethyl eicosapentaenoate (hereinafter, abbreviated as EPA-E).

The drug for use in improving prognosis of the present invention may contain any of other fatty acids as an additional active ingredient together with EPA, not to mention that the pure product of EPA can be used for. Examples of those fatty acids include unsaturated fatty acids such as docosahexaenoic acid, docosapentaenoic acid, docosamonoenic acid, arachidonic acid, eicosatetraenoic acid, eicosatrienoic acid, eicosamonoenic acid octadecatetraenoic acid, α-linolenic acid, linoleic acid, oleic acid, palmitoleic acid, hexadecatetraenoic acid, hexadecatrienoic acid, and hexadecadienoic acid; or saturated acids such as behenic acid, arachidic acid, stearic acid, palmitic acid, and myristic acid.
Furthermore, the exemplified fatty acids described above, in addition to the free forms thereof, each of them may be a salt with an inorganic base such as a sodium salt or a salt with an organic base such as a benzylamine salt, alternatively, an ester form of an alkyl ester such as ethyl ester or glyceride.

In the drug for use in improving prognosis of the present invention, when a fatty acid other than EPA is contained as an active ingredient, it is preferable to contain EPA with a content of 50% or more by weight, preferably 70% or more by weight, more preferably 85% or more by weight in the whole fatty acids. It is preferable that the content of arachidonic acid is small.

As a drug for use in improving prognosis of the present invention, the active ingredient (compound) may be administered without modification. In addition, the active ingredient may be administered after dissolving it in hot water or may be administered in a suspension through a stomach tube. It may be also prepared as a suitable pharmaceutical preparation in advance to administration.
In pharmaceutical preparation, appropriate carriers or media and various additives, which are used generally, can be arbitrarily chosen and then combined so as to be used in combination. For instance, any of excipients, binders, lubricants, colorants, flavors, sterilized water, vegetable oils, harmless organic solvents, harmless solubilizers (e.g., glycerin and propylene glycol), emulsifiers, suspending agents (e.g., Tween 80 and a gum arabic solution), isotonizing agents, pH adjustors, stabilizers,and soothing agents can be used if necessary.

In particular, as the EPA is highly unsaturated, the above pharmaceutical preparation preferably contains an antioxidant in an effective amount enough to prevent the oxidation of EPA. Examples of the antioxidant include butylated hydroxytoluene (BHT), butylated hydroxyanisole (BHA), propyl gallate, gallic acid, and pharmaceutically acceptable quinone and α-tocopherol.

Dosage forms of the pharmaceutical preparations include forms such as tablets, capsules, microcapsules, granules, fine granules, powders, liquid preparations for oral administration, suppositories, syrups, inhalants, eye drops, ointments, and injections (emulsive, suspended, and nonaqueous), or solid injections to be used in an emulsion or a suspension at the time of use.

According to the present invention, the active ingredient can be administered to a patient without modification or as a pharmaceutically prepared drug for use in improving prognosis orally or through a stomach tube.

Of those, it is particularly preferable to administer orally by encapsulating in a capsule such as a soft capsule or a microcapsule.

Since EPA-E-containing soft capsule agent less express side effects and is commercially available as a safe therapeutic drug for arteriosclerosis obliterans and hyperlipemia, the high-purity EPA-E-containing soft capsule agent commercially available in Japan as Epadel and Epadel-S (both manufactured by Mochida pharmaceutical Co., Ltd.) may be used.

The drug for use in improving prognosis of the present invention can be administered at a dose sufficient to express an effect of interest and can be arbitrarily increased or decreased in consideration of the dosage form, administration method, the number of doses per day, the degree of symptoms, weight, and age.

In a case of an oral administration, EPA is administered at a dose of 0.1 to 9 g per day, preferably 0.5 to 6 g per day, more preferably 1 to 3 g per day divided in three times. However, the total dose may be administered at once or in several times if necessary.

When the oral administration is difficult, the above dose of EPA can be dissolved or suspended in hot water and then administered through a stomach tube (Magen Sonde). EPA is preferably administered after a meal. However, in the case of difficulty in orally dietary intake under IVH control, for raising the absorption of EPA, a cholagogue drug such as ursodeoxycholic acid (trade name: Urso, manufactured by Mitsubishi Pharma Corporation, or the like) can be administered in combination. Specifically, for example, there is a drug for use in which 50
mg of Urso granule (manufactured by Mitsubishi Pharma Corporation) is administered through the stomach tube and after 15 minutes 600 mg of EPA previously dissolved or suspended in hot water is then administered through the stomach tube.

In a case of intravenous or intraarterial administration, EPA is administered at a dose of 1-200 mg, preferably 5-100 mg, further preferably 10-50 mg at once or in divided dosages. However, it can be continuously administered over hours to several days using a drip or infusion pump or the like if necessary.

For a dosage period of EPA and the dosage schedule thereof, it is possible to administer for an optional dosage period and an arbitrary dosage schedule from the time after the operation involved in subarachnoid hemorrhage to the time after discharge from a hospital through the length of hospitalization. Pharmaceutical preparations commercially available in the market at present are contraindicated in bleeding patients. Care should be exercised in preoperative and intraoperative administration. From point of view of avoiding the promotion of bleeding tendency, it is preferable to start the administration postoperatively. For a rough standard of the dosage period, it is preferable to continuously administer over 1 week, further preferably from 2 weeks to 1 year.

### Examples

Examples of the present invention will be described below.

### (Example 1)

### [Object and Method]

Sixty four patients with subarachnoid hemorrhage caused by the ruptured aneurysm and subjected to surgical operations by the third day after onset (22 males and 42 females of the ages from 30's-80's) were divided into two groups (each having 32 individuals) designated as an EPA-administration group and an EPA-non-administration group. In addition, no difference was recognized in their background factors of both groups.

Just after the operation, the EPA administration group received the administration of ethyl eicosapentaenoate (trade name: Epadel, manufactured by Mochida Pharmaceutical Co., Ltd.), 1,800 mg divided in three-doses a day for 14 days. Subsequently, the administration was continued during the hospital stay. At the stage of being impossible to dietary intake, tube-feedings were carried out. EPA suspended in hot water was administered through a stomach tube (Magen Sonde). At that time, for enhancing the absorption of EPA, 50 mg of Urso granule (manufactured by Mitsubishi Pharma Corporation) was administered through the stomach tube 15 minutes before EPA administration. At the stage of being possible to dietary intake, a commercial EPA preparation was administered orally. Furthermore, both groups were treated in combination with basic treatments on subarachnoid hemorrhage. However, some of the drugs were defined as prohibited substances.

### [Results]

At the 30th day from the onset of subarachnoid hemorrhage, an evaluation was carried out by the Glasgow Outcome Scale (GOS). The results are listed in Table 1.

**Table 1**

| GOS | GR | MD | SD | VS | D |
|---|---|---|---|---|---|
| EPA-non-administration group | 13 | 4 | 6 | 1 | 8 |
| EPA-administration group | 21 | 6 | 2 | 2 | 1 |

| | | | | | |
|---|---|---|---|---|---|
| GR: Good recovery MD: Moderate disability SD: Severe disability VS: Vegetative state D: Death | | | | | |

As shown in Table 1, the EPA-administration group showed good results. As a result of the Wilcoxon U test, the P value was P=0.0128, resulting in significant difference between both groups.

In addition, the survival rates are listed in Table 2.

**Table 2**

| Survival rate | Death | Survival | Survival rate |
|---|---|---|---|
| EPA-non-administration group | 8 | 24 | 75% |
| EPA-administration group | 1 | 31 | 97% |

As shown in Table 2, it was confirmed that an extremely high survival rate, i.e., an extremely high lifesaving effect, can be obtained in the EPA-administration group. As a result of the Fisher's exact test, the P value obtained was P = 0.0265, and thus a significant difference was found between both groups.

The adverse event considered as a result of the EPA administration was not found as well at all.

From the above description, it was confirmed that EPA improves the prognosis in a subarachnoid hemorrhage patient and has an effect of increasing a recovery rate and a survival rate.

### Industrial Availability

A drug for use in improving prognosis of subarachnoid hemorrhage is characterized by that, as active ingredient, at least one selected from the group consisting of eicosapentaenoic acid and pharmaceutically acceptable salts and esters thereof improves the prognosis in a subarachnoid hemorrhage patient and has an effect of increasing a recovery rate and a survival rate.

## Claims

1. A drug for use in improving the prognosis of a patient with subarachnoid hemorrhage, containing as an active ingredient at least one member selected from the group consisting of eicosapentaenoic acid, pharmaceutically acceptable salts and esters thereof, wherein the drug is administered orally or through a stomach tube, and wherein the term "improving prognosis" designates an improvement in the disease state or lethality of subarachnoid hemorrhage.

2. Use of a drug for the preparation of a medicament for improving the prognosis of a patient with subarachnoid hemorrhage, containing as an active ingredient at least one member selected from the group consisting of eicosapentaenoic acid, pharmaceutically acceptable salts and esters thereof, wherein the drug is administered orally or through a stomach tube, and wherein the term "improving prognosis" designates an improvement in the disease state or lethality of subarachnoid hemorrhage.

3. A drug for use in improving the prognosis of a patient with subarachnoid hemorrhage, containing as an active ingredient at least one member selected from the group consisting of eicosapentaenoic acid, pharmaceutically acceptable salts and esters thereof, wherein the drug is administered orally or through a stomach tube after an operation involved in subarachnoid hemorrhage, and wherein the term "improving prognosis" designates an improvement in the disease state or lethality of subarachnoid hemorrhage.

4. Use of a drug for the preparation of a medicament for improving the prognosis of a patient with subarachnoid hemorrhage, containing as an active ingredient at least one member selected from the group consisting of eicosapentaenoic acid, pharmaceutically acceptable salts and esters thereof, wherein the drug is administered orally or through a stomach tube after an operation involved in subarachnoid hemorrhage, and wherein the term "improving prognosis" designates an improvement in the disease state or lethality of subarachnoid hemorrhage.

5. The drug for use or the use according to any of claims 1 to 4, wherein the improvement in the disease state is an improvement of recovery rate according to the Glasgow Outcome Scale.

6. The drug for use or the use according to any of claims 1 to 4, wherein the drug is administered in combination with a cholagogue drug through a stomach tube.

## Patentansprüche

1. Arzneimittel zur Verwendung bei der Verbesserung der Prognose eines Patienten mit einer Subarachnoidalblutung, das als Wirkstoff mindestens ein Element, ausgewählt aus der Gruppe, bestehend aus Eicosapentaensäure, pharmazeutisch verträglichen Salzen und Estern derselben, enthält, wobei das Arzneimittel oral oder durch eine Magensonde verabreicht wird, und wobei der Begriff "Verbesserung der Prognose" eine Verbesserung des Krankheitszustandes oder der Letalität der Subarachnoidalblutung bezeichnet.

2. Verwendung eines Arzneimittels zur Herstellung eines Medikaments zur Verbesserung der Prognose eines Patienten mit einer Subarachnoidalblutung, das als Wirkstoff mindestens ein Element, ausgewählt aus der Gruppe, bestehend aus Eicosapentaensäure, pharmazeutisch verträglichen Salzen und Estern derselben, enthält, wobei das Arzneimittel oral oder durch eine Magensonde verabreicht wird, und wobei der Begriff "Verbesserung der Prognose" eine Verbesserung des Krankheitszustandes oder der Letalität der Subarachnoidalblutung bezeichnet.

3. Arzneimittel zur Verwendung bei der Verbesserung der Prognose eines Patienten mit einer Subarachnoidalblutung, das als Wirkstoff mindestens ein Element, ausgewählt aus der Gruppe, bestehend aus Eicosapentaensäure, pharmazeutisch verträglichen Salzen und Estern derselben, enthält, wobei das Arzneimittel oral oder durch eine Magensonde nach einer Operation, die eine Subarachnoidalblutung zum Gegenstand hat, verabreicht wird, und wobei der Begriff "Verbesserung der Prognose" eine Verbesserung des Krankheitszustandes oder der Letalität der Subarachnoidalblutung bezeichnet.

4. Verwendung eines Arzneimittels zur Herstellung eines Medikaments zur Verbesserung der Prognose eines Patienten mit einer Subarachnoidalblutung, das als Wirkstoff mindestens ein Element, ausgewählt aus der Gruppe, bestehend aus Eicosapentaensäure, pharmazeutisch verträglichen Salzen und Estern derselben, enthält, wobei das Arzneimittel oral oder durch eine Magensonde nach einer Operation, die eine Subarachnoidalblutung zum Gegenstand hat, verabreicht wird, und wobei der Begriff "Verbesserung der Prognose" eine Verbesserung des Krankheitszustandes oder der Letalität der Subarachnoidalblutung bezeichnet.

5. Arzneimittel zur Verwendung oder Verwendung nach einem der Ansprüche 1 bis 4, wobei die Verbesserung des Krankheitszustands eine Verbesserung der Erholungsrate nach dem Glasgow-Outcome-Score ist.

6. Arzneimittel zur Verwendung oder Verwendung nach einem der Ansprüche 1 bis 4, wobei das Arzneimittel in Verbindung mit einem galletreibenden Arzneimittel durch die Magensonde verabreicht wird.

## Revendications

1. Substance médicamenteuse pour l'utilisation dans l'amélioration du pronostic d'un patient souffrant d'hémorragie sous-arachnoïdienne, contenant comme ingrédient actif au moins un élément sélectionné dans le groupe constitué de l'acide éicosapentaénoïque, de ses sels et esters pharmaceutiquement acceptables, où la substance médicamenteuse est administrée par voie orale ou à travers une sonde gastrique, et où le terme « d'amélioration du pronostic » désigne une amélioration dans l'état pathologique ou dans la létalité de l'hémorragie sous-arachnoïdienne.

2. Utilisation d'une substance médicamenteuse pour la préparation d'un médicament pour l'amélioration du pronostic d'un patient souffrant d'hémorragie sous-arachnoïdienne, contenant comme ingrédient actif au moins un élément sélectionné dans le groupe constitué de l'acide éicosapentaénoïque, de ses sels et esters pharmaceutiquement acceptables, où la substance médicamenteuse est administrée par voie orale ou à travers une sonde gastrique, et où le terme « d'amélioration du pronostic » désigne une amélioration dans l'état pathologique ou dans la létalité de l'hémorragie sous-arachnoïdienne.

3. Substance médicamenteuse pour l'utilisation dans l'amélioration du pronostic d'un patient souffrant d'hémorragie sous-arachnoïdienne, contenant comme ingrédient actif au moins un élément sélectionné dans le groupe constitué de l'acide éicosapentaénoïque, de ses sels et esters pharmaceutiquement acceptables, où la substance médicamenteuse est administrée par voie orale ou à travers une sonde gastrique après une opération impliquée dans l'hémorragie sous-arachnoïdienne, et où le terme « d'amélioration du pronostic » désigne une amélioration dans l'état pathologique ou dans la létalité de l'hémorragie sous-arachnoïdienne.

4. Utilisation d'une substance médicamenteuse pour la préparation d'un médicament pour l'amélioration du pronostic d'un patient souffrant d'hémorragie sous-arachnoïdienne, contenant comme ingrédient actif au moins un élément sélectionné dans le groupe constitué de l'acide éicosapentaénoïque, de ses sels et esters pharmaceutiquement acceptables, où la substance médicamenteuse est administrée par voie orale ou à travers une sonde gastrique après une opération impliquée dans l'hémorragie sous-arachnoïdienne, et où le terme « d'amélioration du pronostic » désigne une amélioration dans l'état pathologique ou dans la létalité de l'hémorragie sous-arachnoïdienne.

5. Substance médicamenteuse pour l'utilisation ou utilisation selon l'une quelconque des revendications 1 à 4, où l'amélioration dans l'état pathologique est une amélioration de la vitesse de récupération selon l'échelle de devenir de Glasgow.

6. Substance médicamenteuse pour l'utilisation ou utilisation selon l'une quelconque des revendications 1 à 4, où la substance médicamenteuse est administrée en combinaison avec un cholagogue à travers une sonde gastrique :
